# EUROPEAN PATENT SPECIFICATION

(11) **EP 0 760 935 B1**
(45) Date of publication and mention of the grant of the patent: **04.10.2000**
(21) Application number: 95921191.3
(22) Date of filing: 17.05.1995
(51) Int. Cl.: G01D 3/10, A61B 5/05, G06F 15/00

(54) **MEASUREMENT SYSTEM AND METHOD**
MESSSYSTEM UND -VERFAHREN
SYSTEME DE MESURE ET PROCEDE ASSOCIE

(30) Priority: 26.05.1994 SE 9401819
(43) Date of publication of application: 12.03.1997
(73) Proprietor: AstraZeneca AB, 151 85 Södertälje (SE)
(72) Inventor: ALDESTAM, Sten, S-226 52 Lund (SE); MARNFELDT, Göran, S-270 35 Blentarp (SE); WALDECK, Johan, S-247 33 Södra Sandby (SE)
(74) Representative: Bjerre, Nils B.J.
(86) International application number: SE9500556
(87) International publication number: WO9533184

(56) References cited:
- EP-B- 0 498 909
- US-A- 5 126 937

## Description

The present invention relates to a measurement system comprising at least one first unit for obtaining measurement values, and a second unit to which each first unit is connected. Furthermore, the invention concerns a method for processing measurement values.

Most prior-art measurement systems comprise one or more sensors, which measure the parameters that are of interest, and a processing unit, which processes the measured values before these are displayed. For instance, US 5 126 937 relates to a biological information measurement system comprising a plurality of measuring sections connected to a processing unit, which converts the values obtained by the measuring sections into output values that are compensated for temperatures measured by the measuring sections, and which outputs the compensated values to a display unit and a recorder.

In many applications, the parameter that is of interest cannot be measured directly, but one or more other parameters have to be measured to enable the parameter that is of interest to be calculated on the basis of the measured values and according to a mathematical model. For instance, if it is desirable to monitor the elasticity of the pulmonary wall in order to study a patient's response to a new drug, the elasticity cannot be measured directly, but the inhalation flow and the pulmonary pressure have to be measured to enable the elasticity to be calculated on the basis thereof.

The mathematical models are sometimes complex, leading to time-consuming calculations which have to be performed in several steps using measurement values from different sensors. To be able to present the results in real time, some steps may have to be carried out in parallel. In a synchronous system, it must then be taken into account that the time for performing the parallel calculation steps may differ. If the system is incorrectly designed and a later calculation step is started before the calculations of the previous steps, whose results are to be used, have been terminated, the time information inherent in the measurement values will be lost and the system will work out of step, yielding incorrect results.

A first object of the present invention is to provide a measurement method and a measurement system eliminating the risk of incorrect results due to calculations performed out of step.

Since the measurement system must take into account the specific time periods required for the different calculations involved, it must be tailored for each specific application, which makes it time-consuming and expensive to use. Incidentally, most measurement systems are used for one application only.

Accordingly, a second object of the present invention is to provide a flexible measurement system, which is easily adapted to different applications.

A further problem related to measurement systems, in particular measurement systems used for research and development, is the documentation of the experiments performed by means of the measurement system. According to good laboratory practice, all pharmacological experiments must be documented in such a way that they can be repeated. However, the primary measurement data and the results of any calculations based thereon are today often stored on a diskette or the like, it being the responsability of the researcher to document how the experiment was performed, which sensors were used with which calibration factors, and so forth. If this information is recorded at all, it is often done manually which may result in errors.

Accordingly, a third object of the present invention is to provide a measurement system facilitating documentation of measurements performed.

The above-mentioned objects are achieved by a measurement system and a method for processing measurement values having the features recited in the appended claims.

Thus, by allocating a time indication to each measurement value and using this time indication to resynchronise values which have been measured at the same time but which may have been subjected to processing of varying duration, there is no longer any risk that time information be lost or that the system works out of step.

In other words, the measurement values which are initially synchronised because they are obtained at the same time can be asynchronously processed and then resynchronised by means of their time indications in order to perform calculations on corresponding measurement values which represent the state of a test object at a specific point of time, to display corresponding measurement values or to perform any other operation which requires synchronised measurement values.

The processing of the measurement values may result in processed measurement values having different time indications from those of the corresponding unprocessed measurement values. However, they are always based on the time indications of the correponding unprocessed measurement values in order to reflect a "time origine" of the processed measurement value. Synchronisation may also involve arranging values in chronological order.

The measurement system according to the invention may comprise one or more first units, e.g. sensor units, for obtaining measurement values. When there is only one first unit, the need for synchronisation of measurement values may arise when the stream of measurement values obtained by the first unit is divided into two identical streams of measurement values which are asynchronously processed. When there are two or more first units by which measurement values are obtained, time indications assigned to measurement values obtained at the same time must be substantially the same to allow later resynchronisation.

The designing of a measurement system according to the present invention is considerably facilitated in comparison of that of a traditional system, since the duration of the different processing steps need not be taken into account.

The system according to the invention is also flexible and easily adapted to different measurements, since all units are intelligent units which include a processor and whose operation is determined by the programs loaded into the different units.

Embodiments of the invention will now be described by way of example with reference to the accompanying drawings, in which
Fig. 1 is a schematic block diagram of the hardware configuration of a measurement system according to the present invention; and
Fig. 2 is a schematic block diagram illustrating the measuring method according to the present invention applied to the measurement of power.

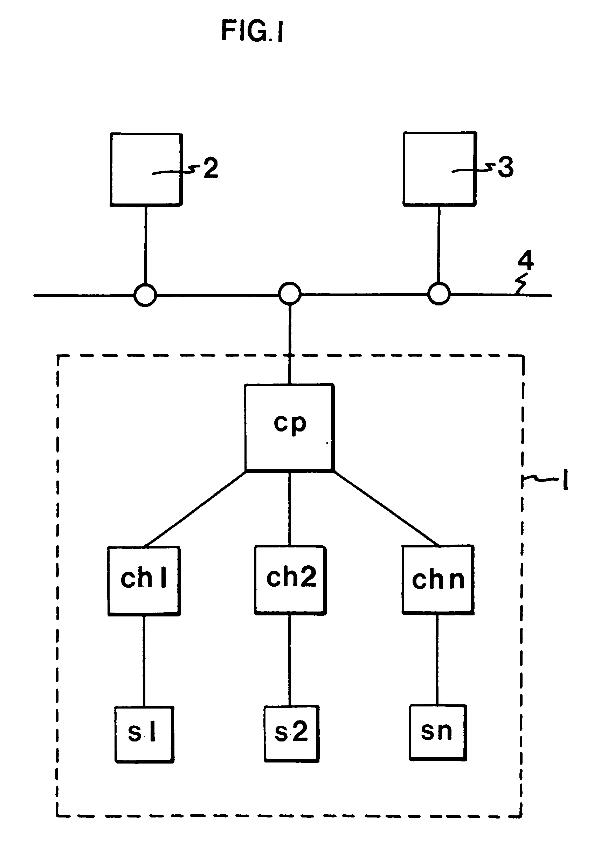

As shown in Fig. 1, a measurement system according to the invention essentially comprises a measurement computer 1, a workstation 2, and a database 3, which all are connected to a data network 4. The measurement computer 1 comprises a plurality of sensor units s1,s2...,sn, each of which is connected to a processing unit cp via respective channel units ch1,ch2...,chn.

When using the measurement system, the sensor units sl-sn are coupled to a test object, e.g. a laboratory animal, for measuring different parameters. To this end, the sensor units may comprise different kinds of gauges, e.g. a pressure gauge, a temperature gauge or a flow gauge.

Furthermore, each sensor unit sl-sn comprises an analog-to-digital converter connected to the gauge, for digitizing the values measured by the gauge, and a processor for processing the measurement values, e.g. scaling them to SI units, and transmitting information to and receiving information from the associated channel unit.

Each channel unit chl-chn comprises a processor which performs calculations in accordance with the programs loaded into the channel unit. More particularly, each channel unit performs the calculations which use only measurement data from the associated sensor unit. As examples of such calculations, filtering and averaging may be mentioned. Also more complex calculations may be performed in the channel units. The purpose of the processing in the channel units is to unload the processing unit by reducing the amount of primary data that it has to process and store. However, if there is no need for unloading the processing unit, the above-mentioned processing by the channel units may be performed by the processing unit.

The channel units ch1-chn are connected to the processing unit cp, e.g. via a common bus. The processing unit comprises a processor which performs calculations requiring measurement data from different sensor units and/or channel units.

Furthermore, each sensor unit sl-sn comprises means for obtaining a time indication for each sampled measurement value. The means may comprise a clock unit, e.g. a register, by means of which time indications can be obtained for the measurement values. More particularly, the processing unit cp comprises a clock, the clock signal of which is used to increment all the clock units which thus are synchronized. When an experiment is started, all the clock units are reset to ensure that they all begin at the same count. Thus, the time indication associated with each measurement value is relative. However, the absolute time of each event in the measurement system may be established if the time of reset of the clock units is stored.

The measurement system operates as follows.

The gauge of each sensor unit sl-sn samples a parameter. The sampled measurement values are analog-to-digital converted and input to the processor. The processor scales the measurement values to SI units and associates with each measurement value a time indication, obtained from the clock unit and representing the time at which the measurement value was sampled, as well as a data stream identity, defining the parameter sampled by the gauge. The thus-created stream of measurement values with associated time indications and data stream identities is transmitted to the channel unit.

The clock units may be arranged in the channel units instead of the sensor units. In this case, the measurement values are forwarded directly to the respective channel units, where the time indication is added.

Each channel unit chl-chn performs the calculations which require only measurement values from the associated sensor unit, e.g. filtering, by means of calculation routines. The measurement values output from each of the calculation routines constitute a stream of processed measurement values. The processor of the channel unit adds a time indication to each of the processed measurement values in the output stream. The purpose of this time indication is to indicate a "time origin" of the processed measurement value so that processed measurement values originating from one and the same sampled measurement value, or from several measurement values sampled at the same time by different sensor units, can be synchronised later on, also when having been subjected to processing of varying duration so that they have lost their initial inherent synchronisation. The time indication which is associated with a certain output processed measurement value usually is the time indication associated with the corresponding measurement value input to the calculation routine. However, it may also be a time indication, which represents a time between two actual sampling times, e.g. when the calculation is an interpolation, or which is selected among the time indications of a plurality of input measurement values. In any case, the time indication of a processed measurement value is based on the time indication(s) of the corresponding one or more unprocessed measurement values. The means for determining the time indication of the processed measurement value may be a part of the calculation routines.

The processor also adds a data stream identity, which defines the parameter obtained by the calculations, to each of these processed measurement values. The stream of processed measurement values resulting from the processing in the channel unit is transmitted to the processing unit. Also the stream of measurement values input to the channel unit and/or any stream of measurement values resulting from an intermediary calculation step may be transmitted to the processing unit, if needed for the calculation steps performed therein or if to be used as an output from the measurement computer.

Usually, the time needed for the processing in the different channel units chl-chn is not the same. Accordingly, the measurement values received at one time by the processing unit from the different channel units are often unconnected, having different "time origins". The same goes for the measurement values which are received at one time from a single channel unit or sensor unit and are based on the same original measurement values, but which may be the result of asynchronous processing of these values.

To enable calculations based on connected or corresponding measurement values, the processing unit buffers the data streams until all measurement values correspond> ing to each other and required for a specific calculation have been received. It uses the time information to synchronise corresponding measurement values from different data streams.

The final data streams resulting from the calculations in the processing unit are transmitted along with other selected data streams to the workstation 2 for display in real time, the time indications permitting measurement values measured by different sensor units at the same time to be displayed at the same time, and to the database 3 for storage. The stored data include measurement values, either processed or as sampled, together with their associated time indications and their data stream identities.

As appears from the foregoing description, the sensor units, the channel units and the processing unit are all intelligent units, which may be adapted to perform different operations in accordance with the program downloaded into the unit. To this end, each unit in the measurement computer is given a unique identity code, which is stored in a non-volatile memory. When the measurement computer is turned on, the processor of the unit reads the identity code in the non-volatile memory. It then requests its program by transmitting its identity code to the processing unit, which requests the programs from the database, which stores all programs applicable in the measurement computer. When the processing unit receives the programs from the database, it downloads them into the channel units and the sensor units.

In addition to all the programs, the database 3 also stores a description of each unit in the measurement computer, for instance including its identity code, the kind of gauge a sensor unit comprises, its mesurement range, its scaling factors, etc. Furthermore, the data-base may contain a description of all the measurements that may be carried out by the measurement system. Such a description may define which programs are used in the different units of the measurement computer, which data streams are input to and output from the programs, and which data streams are to be displayed at the work-station.

When a measurement is to be carried out, the operator selects the measurement at the workstation 2 by indicating the name of the measurement. The appropriate programs, indicated in the description of the measurement, are then downloaded into the measurement computer 1. The operator also specifies the disposition of the experiment for which the measurement is to be performed, e.g. the doses of a drug to be given to the test object. Furthermore, the operator may calibrate the gauges of the sensor units from the workstation, the calibration factors being automatically stored in the database. Furthermore, all the information entered from the workstation is stored in the data base.

This method has many advantages. First, the operator must carry out the experiment the way he intended. Second, the experiment is adequately documented, since the above-mentioned description of the experiment is stored in the database, so that it can be repeated later on. Furthermore, the measurement system can only be operated from the workstation, which ensures that all steps taken are automatically documented and provided with a time indication. Third, if the wrong sensor unit is connected to a certain channel unit, so that the hardware configuration of the measurement computer does not correspond to the description in the database, the system will discover the erroneous coupling when the sensor unit transmits its identity code to the processing unit when requesting its program.

The operator may also configure the system for new measurements by entering a description of the new configuration at the workstation. The description of the new configuration is stored in the database so that it can be selected for use at a later stage.

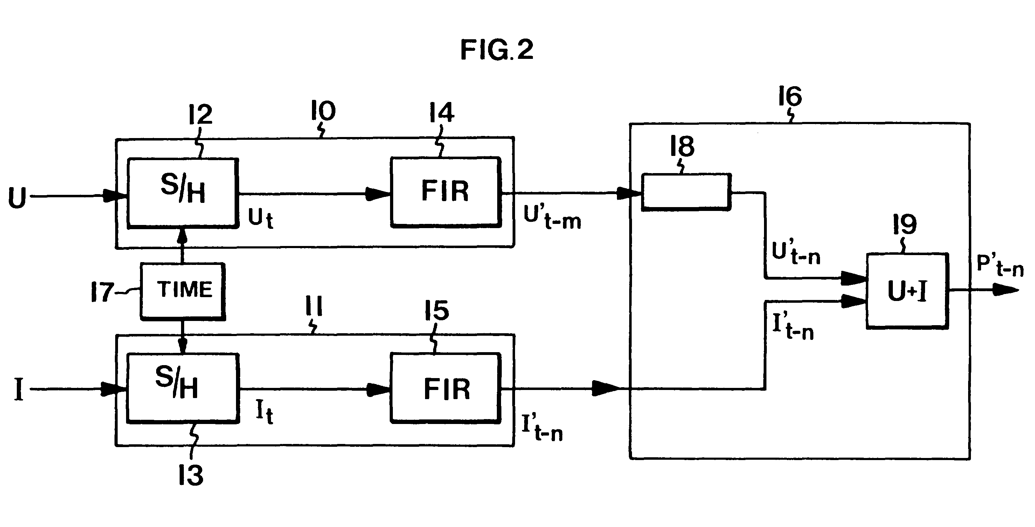

Fig 2 shows an example of how the invention may be applied when measuring power. The measurement system has a first and a second sensor unit 10,11, which each comprises a sample-and-hold circuit 12,13 and a FIR-filter 14,15, as well as a processing unit 16 for calculating the momentary power value. The first sample-and-hold circuit 12 samples a voltage. The voltage value sampled at time t is designated Ut. The sample-and-hold circuit associates the time indication t, obtained from a clock register 17, and a data stream identity "sampled voltage" with the voltage sample Ut. Similairly, the second sample-and-hold circuit 13 samples, at time t, a current I. A time indication t and a data stream identity "sampled current" is associated with the current sample It.

The first FIR filter 14 has a length of 2m. Thus, the output of the FIR filter 14 depends of the values of 2m voltage samples. The filtered voltage value output at time t from the first FIR filter is designated U't-m and a time indication t-m is associated therewith to indicate that it substantially represents the voltage at time t-m. Furthermore, a data stream identity "filtered voltage" is associated with the filtered voltage value.

The second FIR filter 15 has a length of 2n and its output depend of the values of 2n current samples. The filtered current value output at time t by the second FIR filter is designated I't-n. A time indication t-n and a data stream identity "filtered current" are associated with the output. If m is shorter than n, the delay created by the first FIR filter 14 is shorter than that created by the second FIR filter 15. Thus, the filtered voltage value U't-m is available earlier than the corresponding filtered current value I't-m. Therfore, the filtered voltage values are queued in a queue 18 in the processing unit.

At time t, a calculation routine 19 in the processing unit detects that both a filtered voltage value U't-n and a filtered current value I't-n having the same time indication t-n are available and calculates the power Pt-n, and associates the time indication t-n and a data stream identity "power" therewith to indicate that the value is a power value representing the power measured at time t-n.

It goes without saying that the embodiments described above are but examples, which may be modified in many ways within the scope of the appended claims. In the first embodiment described above, the measurement computer comprises n sensor units. However, the invention is applicable to measurement systems which comprise only a single sensor unit, if this sensor unit creates at least two streams of measurement values which need to be synchronised at a later stage in the measurement system. In addition to the processing unit, the synchronisation by means of time indications may be utilised in the sensor units and/or in the channel units as well. Also, the sensor units need not be connected to the processing unit via respective channel units, but may be connected directly to the processing unit, which may perform all the necessary processing.

## Claims

1. A measurement system comprising two first units (s1-sn; ch1-chn) for obtaining a first and a second stream of measurement values, and a second unit (ch1-chn; cp) to which each first unit is connected, **characterised** in that each first unit (s1-sn; ch1-chn) has means for obtaining a time indication, which represents the time at which each measurement value is obtained, and associating said time indication with said measurement value, and that the second unit (ch1-chn; cp) is adapted to use the time indications associated with the measurement values for synchronising measurement values from the first and the second stream, thereby enabling asynchronous processing of the streams before they are received by the second unit.

2. A measurement system according to claim 1, **characterised** in that each first unit comprises processing means for performing such processing of the measurement values obtained by the first unit as is independent of measurement values from the other first units.

3. A measurement system according to claim 2, **characterised** in that each first unit comprises a sensor unit (s1-sn) for obtaining the measurement values and a channel unit (ch1-chn) for performing the processing that is independent of the measurement values from the other first units.

4. A measurement system according to claim 2 or 3, **characterised** in that each first unit (s1-sn; ch1-chn) has means for determining a time indication for each measurement value resulting from the processing performed by the first unit and associating the time indication with the resulting measurement value.

5. A measurement system comprising a first unit (s1-sn; ch1-chn) for obtaining a first stream of measurement values, and a second unit (ch1-chn; cp) to which the first unit is connected, **characterised** in that the first unit (s1-sn; ch1-chn) has means for obtaining a time indication, which represents the time at which each measurement value is obtained, and associating said time indication with said measurement value, as well as means for creating a second stream of measurement value identical with the first stream of measurement values and having the same time indications, and that the second unit (ch1-chn; cp) is adapted to use the time indications associated with the measurement values for synchronising measurement values from the first and second stream, thereby enabling asynchronous processing of the streams before they are received by the second unit.

6. A measurement system according to claim 5, **characterised** in that the first unit comprises processing means for processing the measurement values of at least one of the streams and means for determining a time indication for each measurement value resulting from the processing and associating the time indication with the resulting measurement.

7. A measurement system according to any one of the preceding claims, **characterised** in that said units (s1-sn, ch1-chn, cp) of the system are intelligent units, each including a processor, and that the operation of each unit is determined by a program which is loaded into the unit.

8. A measurement system according to claim 7, **characterised** by a first storage means (3), in which all the programs useable by the different units are stored, said first storage means being connected to the second unit.

9. A measurement system according to any one of the preceding claims, **characterised** in that each of said units (s1-sn, ch1-chn, cp) has a non-volatile memory means storing a unique identity code.

10. A measurement system according to claim 9, **characterised** by a second storage means (3) for storing the unique identity codes together with a description of the properties of the unit identified by said unique identity code, said second storage means being connected to the second unit.

11. A measurement system according to any one of the preceding claims, **characterised** by third storage means (3) for storing the result of the calculations of the processing unit together with a corresponding time indication.

12. A measurement system according to any one of the preceding claims, **characterised** by a synchronising means, which is connected to all the means for obtaining a time indication for the synchronisation thereof.

13. A method for processing measurement values, comprising the step of obtaining at least a first and a second stream of measurement values, **characterised** by the further steps of obtaining, for each measurement value, a time indication, which represents the time at which the measurement value was obtained, and associating it with the measurement value; processing the streams of measurement values asynchronously; and resynchronising measurement values from the first and the second stream by means of the associated time indications.

14. A method for processing measuring values, comprising the step of obtaining a first stream of measurement values, **characterised** by the further steps of obtaining, for each measurement value, a time indication, which represents the time at which the measurement value was obtained, and associating it with the measurement value; creating a second stream of measurement values identical with the first stream of measurement values and having the same time indications; processing the streams of measurement values asynchronously; and resynchronising the measurement values by means of the time indications.

15. A method according to claim 13 or 14, **characterised** in that the step of processing the streams of measurement values asynchronously comprises the steps of processing at least one of the streams of measurement values, determining a time indication for each processed measurement value and associating it with the measurement value.

16. A method according to any one of claims 13-15, **characterised** in that the time indication for each processed measurement value is determined on the basis of the time indication(s) of the corresponding one or more unprocessed measurement values.

17. A method according to any one of claims 13-16, **characterised** in that at least the first stream of measurement values is obtained by sampling a measurement parameter of a test object.

18. A method according to any one of claims 13-17, **characterised** by the further step of associating, with each measurement value, a stream identity, which indicates the parameter represented by the measurement value.

## Patentansprüche

1. Meßsystem mit zwei ersten Einheiten (s1-sn; ch1-chn) zum Empfangen eines ersten und eines zweiten Stromes von Meßwerten und einer zweiten Einheit (ch1-chn; cp), mit welcher jede erste Einheit verbunden ist, dadurch gekennzeichnet, daß jede erste Einheit (s1-sn; ch1-chn) eine Einrichtung zum Empfangen einer Zeitangabe, welche die Zeit wiedergibt, zu welcher jeder Meßwert erhalten wird, und zur Zuordnung der Zeitangabe zu dem Meßwert aufweist und daß die zweite Einheit (ch1-chn; cp) für eine Verwendung der den Meßwerten zugeordneten Zeitangaben zur Synchronisierung von Meßwerten aus dem ersten und dem zweiten Strom ausgelegt ist, wobei sie eine asynchrone Verarbeitung der Ströme ermöglicht, bevor diese von der zweiten Einheit empfangen werden.

2. Meßsystem nach Anspruch 1, dadurch gekennzeichnet, daß jede erste Einheit eine Verarbeitungseinrichtung zur Durchführung einer solchen Verarbeitung der Meßwerte, die von der ersten Einheit erhalten wurden, unabhängig von Meßwerten von den anderen ersten Einheiten aufweist.

3. Meßsystem nach Anspruch 2, dadurch gekennzeichnet, daß jede erste Einheit eine Sensoreinheit (s1-sn) zum Empfangen der Meßwerte und eine Kanaleinheit (ch1-chn) zur Durchführung der Verarbeitung, welche von den Meßwerten von den anderen ersten Einheiten unabhängig ist, aufweist.

4. Meßsystem nach einem der Ansprüche 2 oder 3, dadurch gekennzeichnet, daß jede erste Einheit (s1-sn; ch1-chn) eine Einrichtung zur Bestimmung einer Zeitangabe für jeden Meßwert, der aus der von der ersten Einheit durchgeführten Verarbeitung resultiert, und zur Zuordnung der Zeitangabe zu dem resultierenden Meßwert aufweist.

5. Meßsystem mit einer ersten Einheit (s1-sn; ch1-chn) zum Empfangen eines ersten Stromes von Meßwerten und einer zweiten Einheit (ch1-chn; cp), mit der die erste Einheit verbunden ist, dadurch gekennzeichnet, daß die erste Einheit (s1-sn; ch1-chn) eine Einrichtung zur Empfangen einer Zeitangabe, welche die Zeit wiedergibt, zu der jeder Meßwert erhalten wird, und zur Zuordnung dieser Zeitangabe zu dem Meßwert sowie eine Einrichtung zur Erzeugung eines zweiten Stromes von Meßwerten, der mit dem ersten Strom von Meßwerten identisch ist und die gleichen Zeitangaben hat, aufweist und daß die zweite Einheit (ch1-chn; cp) für eine Verwendung der den Meßwerten zugeordneten Zeitangaben zur Synchronisierung von Meßwerten aus dem ersten und dem zweiten Strom ausgelegt ist, wobei eine asynchrone Verarbeitung der Ströme möglich ist, bevor diese von der zweiten Einheit empfangen werden.

6. Meßsystem nach Anspruch 5, dadurch gekennzeichnet, daß die erste Einheit eine Verarbeitungseinrichtung zur Verarbeitung der Meßwerte von wenigstens einem der Ströme und eine Einrichtung zur Bestimmung einer Zeitangabe für jeden Meßwert, der aus der Verarbeitung resultiert, und zur Zuordnung der Zeitangabe zu der resultierenden Messung aufweist.

7. Meßsystem nach einem der vorangegangenen Ansprüche, dadurch gekennzeichnet, daß die Einheiten (s1-sn, ch1-chn, cp) des Systems intelligente Einheiten sind, von denen jede einen Prozessor aufweist, und daß der Betrieb jeder Einheit von einem Programm, das in die Einheit geladen ist, bestimmt wird.

8. Meßsystem nach Anspruch 7, gekennzeichnet durch eine erste Speichereinrichtung (3), in welcher alle von den verschiedenen Einheiten verwendbaren Programme gespeichert werden, wobei diese erste Speichereinrichtung mit der zweiten Einheit verbunden ist.

9. Meßsystem nach einem der vorangegangenen Ansprüche, dadurch gekennzeichnet, daß jede der Einheiten (s1-sn, ch1-chn, cp) einen permanenten Speicher hat, der einen eindeutigen Identitätscode speichert.

10. Meßsystem nach Anspruch 9, gekennzeichnet durch eine zweite Speichereinrichtung (3) zur Speicherung des eindeutigen Identitätscodes zusammen mit einer Beschreibung der Eigenschaften der Einheit, welche durch den eindeutigen Identitätscode identifiziert wird, wobei diese zweite Speichereinrichtung mit der zweiten Einheit verbunden ist.

11. Meßsystem nach einem der vorangegangenen Ansprüche, gekennzeichnet durch eine dritte Speichereinrichtung (3) zur Speicherung des Ergebnisses der Berechnungen der Verarbeitungseinheit zusammen mit einer entsprechenden Zeitangabe.

12. Meßsystem nach einem der vorangegangenen Ansprüche, gekennzeichnet durch eine Synchronisierungseinrichtung, welche mit allen Einrichtungen zum Empfang einer Zeitangabe für die Synchronisierung derselben verbunden ist.

13. Verfahren zur Verarbeitung von Meßwerten mit der Stufe, in der man wenigstens einen ersten und einen zweiten Strom von Meßwerten erhält, gekennzeichnet durch die weiteren Stufen, in denen man für jeden Meßwert eine Zeitangabe erhält, welche die Zeit wiedergibt, zu welcher der Meßwert erhalten wurde, und diese dem Meßwert zuordnet, die Ströme von Meßwerten asynchron verarbeitet und Meßwerte aus dem ersten und dem zweiten Strom mittels der zugeordneten Zeitangaben wieder synchronisiert.

14. Verfahren zur Verarbeitung von Meßwerten mit der Stufe, in der man einen ersten Strom von Meßwerten erhält, gekennzeichnet durch die weiteren Stufen, in denen man für jeden Meßwert eine Zeitangabe erhält, welche die Zeit wiedergibt, zu welcher der Meßwert erhalten wurde, und diese dem Meßwert zuordnet, einen zweiten Strom von Meßwerten erzeugt, der mit dem ersten Strom von Meßwerten identisch ist und die gleichen Zeitangaben hat, die Ströme von Meßwerten asynchron verarbeitet und die Meßwerte mittels der Zeitangaben wieder synchronisiert.

15. Verfahren nach einem der Ansprüche 13 oder 14, dadurch gekennzeichnet, daß die Stufe der asynchronen Verarbeitung der Ströme von Meßwerten die Stufen umfaßt, in denen man wenigstens einen der Ströme von Meßwerten verarbeitet, für jeden verarbeiteten Meßwert eine Zeitangabe bestimmt und diese dem Meßwert zuordnet.

16. Verfahren nach einem der Ansprüche 13 bis 15, dadurch gekennzeichnet, daß die Zeitangabe für jeden verarbeiteten Meßwert auf der Grundlage der Zeitangabe(n) des/der entsprechenden einen oder mehreren nicht verarbeiteten Meßwerte(s) bestimmt.

17. Verfahren nach einem der Ansprüche 13 bis 16, dadurch gekennzeichnet, daß wenigstens der erste Strom von Meßwerten durch Abtastung eines Meßparameters eines Testobjekts erhalten wird.

18. Verfahren nach einem der Ansprüche 13 bis 17, gekennzeichnet durch die weitere Stufe, in der man jedem Meßwert eine Stromidentität zuordnet, welche den von dem Meßwert wiedergegebenen Parameter angibt.

## Revendications

1. Dispositif de mesure comprenant deux premières unités (s1-sn; ch1-chn) destinées à obtenir un premier et second flux de valeurs de mesure, et une seconde unité (ch1-chn ; cp) à laquelle chaque première unité est reliée, caractérisé en ce que chaque première unité (s1-sn ; ch1-chn) comporte un moyen destiné à obtenir une indication de temps, qui représente l'instant où est obtenue chaque valeur de mesure, et à associer ladite indication de temps à ladite valeur de mesure, et en ce que la seconde unité (ch1-chn ; cp) est conçue de manière à utiliser les indications de temps associées aux valeurs de mesure afin de synchroniser les valeurs de mesure provenant des premier et second flux, permettant ainsi le traitement asynchrone des flux avant qu'ils ne soient reçus par la seconde unité.

2. Dispositif de mesure selon la revendication 1, caractérisé en ce que chaque première unité comprend un moyen de traitement destiné à exécuter un tel traitement des valeurs de mesure obtenues par la première unité de manière indépendante des valeurs de mesure à partir des autres premières unités.

3. Dispositif de mesure selon la revendication 2, caractérisé en ce que chaque première unité comprend une unité de détection (s1-sn) destinée à obtenir les valeurs de mesure et une unité formant voie de mesure (ch1-chn) destinée à exécuter le traitement qui est indépendant des valeurs de mesure à partir des autres premières unités.

4. Dispositif de mesure selon la revendication 2 ou 3, caractérisé en ce que chaque première unité (s1-sn ; ch1-chn) comprend un moyen destiné à déterminer une indication de temps pour chaque valeur de mesure provenant du traitement réalisé par la première unité et à associer l'indication de temps à la valeur de mesure résultante.

5. Dispositif de mesure comprenant une première unité (s1-sn ; ch1-chn) destinée à obtenir un premier flux de valeurs de mesure, et une seconde unité (ch1-chn ; cp) à laquelle la première unité est reliée, caractérisé en ce que la première unité (s1-sn ; ch1-chn) comporte un moyen destiné à obtenir une indication de temps, qui représente l'instant où est obtenue chaque valeur de mesure, et à associer ladite indication de temps à ladite valeur de mesure, de même qu'un moyen destiné à créer un second flux de valeurs de mesure identique au premier flux de valeurs de mesure et comportant les mêmes indications de temps, et en ce que la seconde unité (ch1-chn ; cp) est conçue de manière à utiliser les indications de temps associées aux valeurs de mesure afin de synchroniser les valeurs de mesure provenant des premier et second flux, permettant ainsi le traitement asynchrone des flux avant qu'ils ne soient reçus par la seconde unité.

6. Dispositif de mesure selon la revendication 5, caractérisé en ce que la première unité comprend un moyen de traitement destiné à traiter les valeurs de mesure d'au moins un des flux et un moyen destiné à déterminer une indication de temps pour chaque valeur de mesure résultant du traitement et à associer l'indication de temps à la mesure résultante.

7. Dispositif de mesure selon l'une quelconque des revendications précédentes, caractérisé en ce que lesdites unités (s1-sn ; ch1-chn, cp) du dispositif sont des unités intelligentes comprenant chacune une unité de traitement, et en ce que le fonctionnement de chaque unité est déterminé par un programme qui est chargé dans l'unité.

8. Dispositif de mesure selon la revendication 7, caractérisé par un premier moyen de mémorisation (3), dans lequel tous les programmes pouvant être utilisés par les différentes unités sont mémorisés, ledit premier moyen de mémorisation étant relié à la seconde unité.

9. Dispositif de mesure selon l'une quelconque des revendications précédentes, caractérisé en ce que chacune desdites unités (s1-sn ; ch1-chn, cp) comprend un moyen formant mémoire non volatile destiné à mémoriser un code d'identité unique.

10. Dispositif de mesure selon la revendication 9, caractérisé par un deuxième moyen de mémorisation (3) destiné à mémoriser les codes d'identité uniques simultanément à une description des propriétés de l'unité identifiée par ledit code d'identité unique, ledit deuxième moyen de mémorisation étant relié à la seconde unité.

11. Dispositif de mesure selon l'une quelconque des revendications précédentes, caractérisé par un troisième moyen de mémorisation (3) destiné à mémoriser le résultat des calculs de l'unité de traitement simultanément à une indication de temps correspondante.

12. Dispositif de mesure selon l'une quelconque des revendications précédentes, caractérisé par un moyen de synchronisation, qui est relié à l'ensemble des moyens destinés à obtenir une indication de temps afin d'assurer leur synchronisation.

13. Procédé de traitement de valeurs de mesure, comprenant l'étape d'obtention d'au moins'un premier et un second flux de valeurs de mesure, caractérisé par les étapes supplémentaires d'obtention, pour chaque valeur de mesure, d'une indication de temps, qui représente l'instant où la valeur de mesure a été obtenue, et d'association de celle-ci à la valeur de mesure ; de traitement des flux des valeurs de mesure de manière asynchrone, et de remise en synchronisation des valeurs de mesure à partir des premier et second flux au moyen des indications de temps associées.

14. Procédé de traitement de valeurs de mesure, comprenant l'étape d'obtention d'un premier flux de valeurs de mesure, caractérisé par les étapes supplémentaires d'obtention, pour chaque valeur de mesure, d'une indication de temps, qui représente l'instant où la valeur de mesure a été obtenue, et d'association de celle-ci à la valeur de mesure ; de création d'un second flux des valeurs de mesure identique au premier flux des valeurs de mesure et présentant les mêmes indications de temps ; de traitement des flux des valeurs de mesure de manière asynchrone, et de remise en synchronisation des valeurs de mesure au moyen des indications de temps.

15. Procédé selon la revendication 13 ou 14, caractérisé en ce que l'étape de traitement des flux des valeurs de mesure de manière asynchrone comprend les étapes de traitement d'au moins un des flux des valeurs de mesure, de détermination d'une indication de temps pour chaque valeur de mesure traitée et d'association de celle-ci à la valeur de mesure.

16. Procédé selon l'une quelconque des revendications 13 à 15, caractérisé en ce que l'indication de temps pour chaque valeur de mesure traitée est déterminée en fonction des indications de temps des valeurs de mesure non traitées correspondantes.

17. Procédé selon l'une quelconque des revendications 13 à 16, caractérisé en ce qu'au moins le premier flux des valeurs de mesure est obtenu par échantillonnage d'un paramètre de mesure d'un objet éprouvé.

18. Procédé selon l'une quelconque des revendications 13 à 17, caractérisé par l'étape supplémentaire d'association, à chaque valeur de mesure, d'une identité de flux, qui indique le paramètre représenté par la valeur de mesure.
